# EUROPEAN PATENT SPECIFICATION

(11) **EP 4 220 085 B1**
(45) Date of publication and mention of the grant of the patent: **21.10.2026**
(21) Application number: 23161080.9
(22) Date of filing: 11.06.2021
(51) Int. Cl.: G01C 21/14, A61B 5/11, G01C 21/20, G01C 22/00

(54) **MEASURING METHOD AND DEVICE**
MESSVERFAHREN UND -VORRICHTUNG
PROCÉDÉ ET DISPOSITIF DE MESURE

(30) Priority: 12.06.2020 US 202016900618
(43) Date of publication of application: 02.08.2023
(62) Document number(s) of the earlier application(s) in accordance with Art. 76 EPC: 21752716.7 / 4 165 371
(73) Proprietor: Koneksa Health Inc., New York, NY 10007 (US)
(72) Inventor: KELLY, Peter John, London, EC4A 3TW (GB); ELLIS, Robert David, New York, 10007 (US); HUANG, Chengrui, New York, 10007 (US)
(74) Representative: MacDougall, Alan John Shaw

(56) References cited:
- WO-A1-2015/113915
- US-A1- 2018 192 917
- US-A1- 2018 264 320

## Description

### Field of the invention

The present invention relates to the field of measurement of movement of a user. The invention has particular although not exclusive relevance to methods and devices for measuring and/or analysing the movement of a user to determine the step and stride periods of a user.

### Background

Devices such as activity monitors or pedometers are used to measure movements of a user. These devices can be used to determine when a user is walking and from this, the number of steps, or step count, of the user can also be determined.

Current devices are typically aimed at the leisure market - where accuracy is less important than repeatability. The devices may be dedicated devices designed to monitor the user's steps or they may take the form of a software application running on a user device such as a mobile (cellular) telephone or a smart watch or the like. As anyone who has used these devices will be aware, the different devices often give very different step counts even though the distance walked may be the same.

Existing methods and devices analyse the magnitude of the data generated by an accelerometer mounted in the user's device as the user is walking. Specifically, existing methods and devices typically either calculate the auto-correlation of this magnitude data over time periods or more crudely detect the spikes in the magnitude data corresponding to heel strikes, to work out periodic motions corresponding to the user's steps which are then counted. However, these analyses also capture other periodic motions such as the user's stride period (the period between a first heel striking the ground and the next time the first heel strikes the ground) which should be about twice the step period (the time interval between the first heel striking the ground and the second heel striking the ground). Typically, a user's step period when walking is less than about 0.8 seconds, and existing techniques typically compare the determined periods with this threshold in order to try to differentiate between step periods and stride periods. However, the inventors have realised that the existing methods, including this thresholding leads to errors in the calculations that are made.

A need exists for devices and methods that can determine more accurately the movements of the user. Such devices and methods can of course be used in the leisure market, where users will appreciate the more accurate information, but they can also help to open up new markets for this kind of analysis. For example, athletes are always looking for devices and methods that can accurately analyse their movements to allow them to improve their technique and gain an advantage over their competitors. Devices that are able to track and accurately monitor movements of the user can also be used in the medical field either for remote diagnosis purposes or for collecting data that may be relevant to a clinical study. For example, the movement information may be required for correlation with other sensors and time-specific measurements and the absence of this data may have a negative impact on determining the efficacy of therapies. For medical applications the requirement for accuracy is particularly important as it may affect treatment decisions and/or results of drug trials, which may have serious health consequences.

Some medical conditions, such as central nervous system disorders, may result in a subject having an atypical style of walking, so devices and algorithms optimised for the general (i.e. healthy) population may be inappropriate.

Some subjects may be unable or unwilling to wear a device in a specific position (e.g. ankle) or a specific device (e.g. a watch), so the device and algorithm should ideally be agnostic as regards to wear position and should be valid across a variety of hardware devices to accommodate these difficulties.

WO 2015/113915 discloses a method of processing measurements of acceleration to identify steps by a user, the method comprising obtaining measurements of acceleration from a device worn or carried by a user; analysing the measurements of acceleration to determine a value for a threshold that is to be used to identify steps by the user in measurements of acceleration; and using the determined value for the threshold to identify steps by the user in measurements of acceleration.

US 2018/0192917 discloses a method of determining the orientation of an accelerometer that is being carried or worn by a user, the method comprising measuring acceleration using the accelerometer as the user moves; processing the measured acceleration to determine a step frequency of the movements by the user; and processing the measured acceleration to identify a lateral component of acceleration as a component of acceleration having a high response at a frequency corresponding to half the step frequency.

US 2018/0264320 discloses a system and method for automatic location detection for wearable sensors. The method includes collecting kinematic data from at least one kinematic activity sensor coupled to a user; generating a set of base kinematic metrics; assessing a set of sensor state discriminators and identifying a kinematic monitoring mode; and activating the kinematic monitoring mode at a kinematic activity sensor.

### Summary

The present invention provides an apparatus and a method in accordance with the independent claims. Other aspects of the invention are provided herein in accordance with the dependent claims.

According to a first aspect there is provided an apparatus and method for determining movement information for a user that carries an accelerometer whilst moving, which do not form part of the invention. The apparatus receives acceleration data from the accelerometer that are defined relative to a frame of reference of the accelerometer. A transformation is determined and applied to the acceleration data or to data derived from the acceleration data to determine acceleration data in a frame of reference of the user that includes a direction of travel of the user and a side to side direction transverse to the direction of travel of the user. The acceleration data or data derived from the acceleration data is analysed to determine a time period corresponding either to a stride period or to a step period of the user as the user is walking or running; and information about accelerations in the side to side direction are used to disambiguate whether the determined time period corresponds to the stride period of the user or to the step period of the user.

In some embodiments, the processor and memory are configured to use information about accelerations in said side to side direction and in said direction of travel to disambiguate whether the determined time period corresponds to the stride period of the user or to the step period of the user.

The processor and memory may determine a first autocorrelation function to determine said time period corresponding either to said stride period or to said step period of the user and may process the first autocorrelation function to identify a peak in the first autocorrelation function at an autocorrelation lag corresponding to the stride period of the user or to the step period of the user. In some embodiment, the processor and memory process the first autocorrelation function to identify the highest peak in the first autocorrelation function after a zero lag peak and determine the time period corresponding to the stride period of the user or to the step period of the user as the autocorrelation lag associated with the identified highest peak.

Typically, the processor and memory determine a second autocorrelation function of the accelerations in said side to side direction and disambiguate whether the time period corresponds to the stride period or the step period in dependence upon whether or not the second autocorrelation function includes a peak around the autocorrelation lag corresponding to the step or stride period.

A second autocorrelation function of the accelerations in said side to side direction and a third autocorrelation function of the accelerations in said direction of travel may be determined and used to disambiguate whether the time period corresponds to the stride period or the step period in dependence upon whether or not the second and autocorrelation function includes a peak around the autocorrelation lag corresponding to the step or stride period. The first, second and third autocorrelation functions may also be used to confirm that the user is walking or not walking.

The first autocorrelation function is calculated on said accelerometer data or on transformed accelerometer data that defines accelerations in the user frame of reference.

In one embodiment, the processor and memory are configured to determine and apply a first transformation that aligns a first axis of the accelerometer data or data derived from the accelerometer data with a vertical axis and a second transformation that aligns a second axis of the accelerometer data or data derived from the accelerometer data with said direction of travel and a third axis of the accelerometer data or data derived from the accelerometer data with said side to side direction. These transformations usually comprise a rotation.

In one embodiment, the processor and memory are configured to: determine that the determined time period corresponds to a stride period of the user when the information about accelerations in the side to side direction matches information about accelerations in the direction of travel; and determine that the determined time period corresponds to a step period of the user when the information about accelerations in the side to side direction does not match the information about accelerations in the direction of travel.

The frame of reference of the user usually comprises a vertical direction transverse to both the direction of travel and the side to side direction.

In one embodiment, the processor and memory are configured to process the acceleration data to identify periods of walking within the acceleration data and are configured to determine said time period corresponding either to a stride period or to a step period of the user using acceleration data from within an identified period of walking.

The direction of travel and the side to side direction may be identified as directions in a horizontal plane that have the most variability and the least variability in the received acceleration data. Alternatively, a compass or global positioning system (e.g. GPS) mounted in the user's device may provide direction of travel information.

The processor and memory may be configured to use the disambiguated step period or stride period to determine a step count of the user for movements corresponding to walking or running. This step count information may be stored and/or output to the user (e.g. on a display of the user device). The step count information may also be transmitted to a remote computer.

According to another aspect not forming part of the invention, there is provided an apparatus for determining movement information for a user that carries an accelerometer whilst moving, the apparatus comprising one or more processors and memory configured to: receive acceleration data from the accelerometer, the acceleration data defining accelerations experienced by the accelerometer resulting from movement of the user, the accelerations being defined relative to a frame of reference associated with the accelerometer; apply to the acceleration data or to data derived from the acceleration data a transformation for transforming the frame of reference to a frame of reference of the user that includes a direction of travel of the user and a side to side direction transverse to the direction of travel of the user; determine a first autocorrelation function of the acceleration data or data derived from the acceleration data; determine a second autocorrelation function of accelerations in said direction of travel; determine a third autocorrelation function of accelerations in said side to side direction; and determine if the user is walking or not walking using the first, second and third autocorrelation functions.

There is also provided an apparatus not forming part of the invention, for determining movement information for a user that carries an accelerometer whilst moving, the apparatus comprising one or more processors and memory configured to: receive acceleration data from the accelerometer, the acceleration data including for each of a plurality of time points, acceleration values for a first plurality of orthogonal directions defined by an orientation of the accelerometer, each acceleration value representing acceleration of the accelerometer in one of the first plurality of orthogonal directions at a given time point; transform the acceleration data to transformed acceleration data that includes for each of the plurality of time points, acceleration values for a second plurality of orthogonal directions defined by an orientation of the user, each acceleration value representing acceleration movements of the accelerometer in one of the second plurality of orthogonal directions, the second plurality of orthogonal directions including a direction of travel of the user and a side to side direction transverse to the direction of travel of the user; analyse the acceleration data or at least part of the transformed acceleration data to determine a time period corresponding either to a stride period or a step period of the user; and use the transformed acceleration data relating to movements of the user in at least said side to side direction to disambiguate whether the determined period corresponds to the stride period of the user or to the step period of the user.

The apparatus summarised above may form part of a user device (such as a mobile (cellular) telephone, a smart watch or the like) carried by the user and the accelerometer may form part of the user device or may be in a separate device that communicates with the user device. The apparatus summarised above may also form part of a central server that receives acceleration data from the user device and that processes the received acceleration data to determine the movement information.

One aspect not forming part of the invention also provides a method for determining movement information for a user that carries an accelerometer whilst moving, the method comprising: receiving acceleration data from the accelerometer, the acceleration data defining accelerations experienced by the accelerometer resulting from movement of the user, the accelerations being defined relative to a frame of reference associated with the accelerometer; applying to the acceleration data or to data derived from the acceleration data a transformation for transforming the frame of reference to a frame of reference of the user that includes a direction of travel of the user and a side to side direction transverse to the direction of travel of the user; analysing the acceleration data or data derived from the acceleration data to determine a time period corresponding either to a stride period or to a step period of the user as the user is walking or running; and using information about accelerations in said side to side direction to disambiguate whether the determined time period corresponds to the stride period of the user or to the step period of the user.

Another aspect not forming part of the invention also provides a computer program product (which may be a tangible computer readable medium or a carrier signal) comprising computer implementable instructions for causing a programmable computer device to become configured as the apparatus summarised above.

Another aspect not forming part of the invention also provides a clinical trial system and method comprising a central computer that communicates with a plurality of user devices, each user device being arranged to collect acceleration data relating to movement of the user associated with the user device; and wherein the central computer or at least one user device comprises an apparatus as summarised above.

### Brief Description of the Drawings

Exemplary embodiments of the invention will now be described with reference to the accompanying figures in which:
Figure 1A schematically illustrates a clinical trial in which the movement of users taking part in the trial is determined by user devices worn or carried by the users and reported to a central server for collection and analysis;
Figure 1B is a block diagram illustrating the main electronic parts of the system shown in Figure 1A;
Figure 2 is a block diagram illustrating the main components of a user device shown in Figure 1B;
Figure 3 is a flow diagram illustrating a prior art technique for determining the step or stride period of a user;
Figure 4 is a plot illustrating an autocorrelation function calculated from the accelerometer data obtained whilst the user is walking;
Figure 5 is a flow diagram illustrating a preferred technique for determining and disambiguating the step and stride period of a user whilst walking;
Figure 6 is a plot illustrating autocorrelations calculated from the accelerometer data obtained whilst the user is walking and used to disambiguate step and stride periods; and
Figure 7 illustrates a flow diagram illustrating a preferred way for determining whether a period of movement corresponds to a period of walking or not walking.

In the drawings, like reference numerals are used to indicate like elements.

### Detailed Description

### Overview

As summarised above, the invention provides alternative ways for analysing a user's movements. The methods and devices provided by the invention can be used in various applications, such as in fitness trackers and the like. However, the invention can also be used in a medical setting which will now be described.

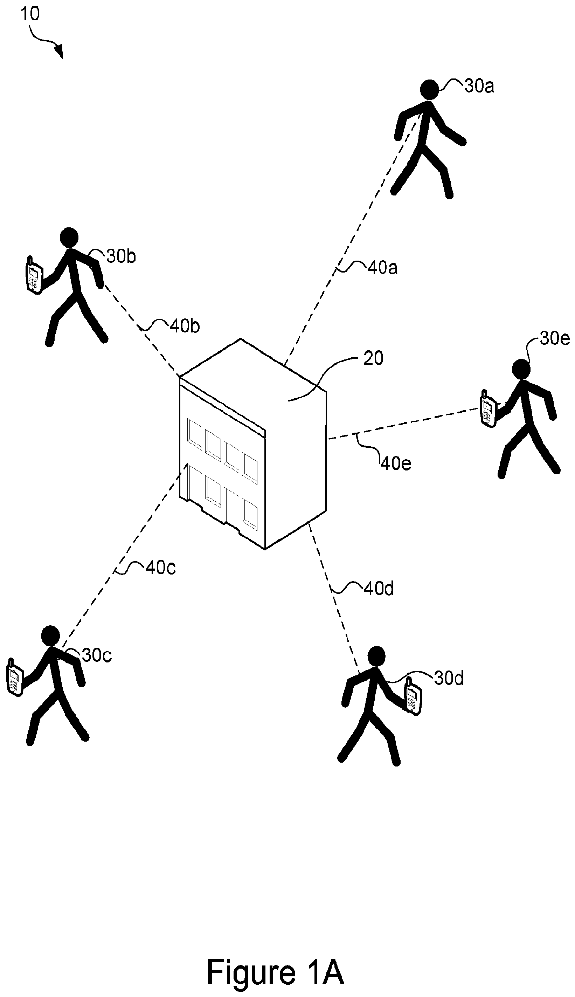

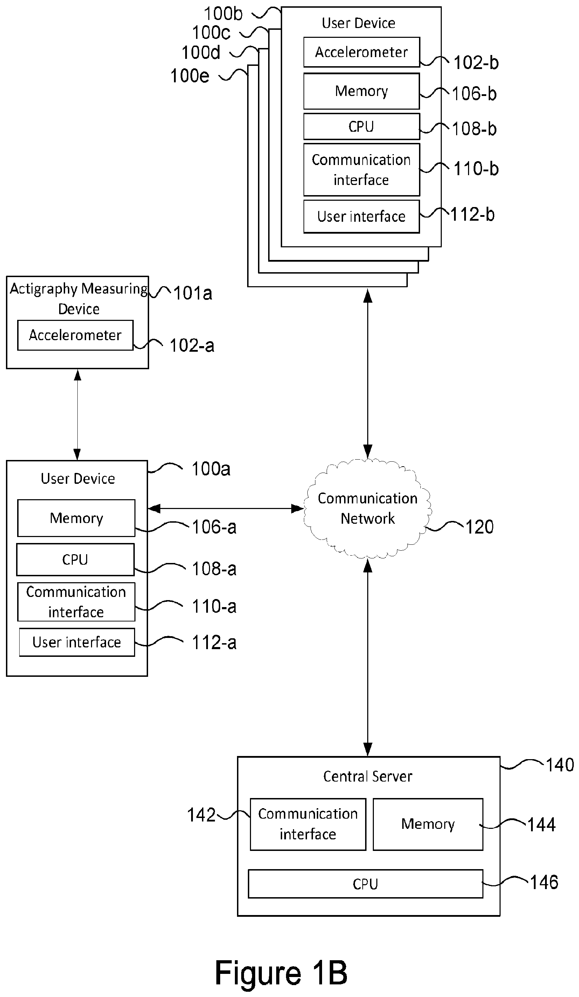

More specifically, Figures 1A and 1B illustrate how the invention can be used in a clinical trial system 10 in which a number of subjects (also referred to below as users) 30a to 30e use a respective user device 100a to 100e to monitor the movements of the corresponding subject when they are walking. The information gathered by the user devices 100 is transmitted over a communication network 120 (represented in Figure 1A by the broken lines 40a to 40e) back to a central server 140 with results that may be displayed within the clinic 20.

The clinic 20 may be a health centre such as a hospital or doctor's surgery. It may comprise a single centre or a number of centres located in a number of different geographical locations. The subjects 30a-30e are patients of the clinic 20 and are taking part in a medical trial, organised by the clinic 20. Each of the patients in the medical trial are cohorted into groups with the same medical condition.

Each of the subjects 30a-30e is provided with a user device 100 that may be dedicated to the clinic and returned to the clinic after the trial is over. Alternatively, the clinic may provide the subject with a software application that they can run on their own user device - such as a cellular telephone or a smart watch or the like. In either case, each subject is asked to wear or carry their user device so that an accelerometer associated with the user device can capture the movements of the user during the clinical trial. As shown in Figure 1B, some user devices 100 have a built-in accelerometer 102 but some (in this example user device 100a) do not. Where the user device 100 does not have an accelerometer, a separate actigraphy measuring device 101 is provided that has an accelerometer 102-a for capturing the movements of the subject 30a. The actigraphy measuring device 101 is worn or carried by the subject, for example, around the subject's wrist, ankle, in a pocket, on a belt, held in a hand, placed in a bag worn by the subject or worn as a pendant, for example around the subject's neck.

Accelerometers typically provide acceleration information in three orthogonal directions which depend on the orientation of the accelerometer. By analysing the accelerometer data, the user device 100 can determine movement information about the subject which is then transmitted (wirelessly or over a wired connection) as subject data to the central server 140 for further analysis as part of the medical trial.

In one example, the subject data provided to the central server 140 comprises walking data and identification data that identifies the subject to which the walking data relates. The walking data may comprise one or more of: step count, walking or activity periods, and distance walked, over a period specified by the trial, for example a day, week, month or year. The subject data may be retrieved from the user device 100 when the subject visits the clinic, or the subject data may be transmitted to the clinic over a cellular or wired telephone or computer network (wirelessly or over a wired connection). Subject data collected at the clinic can be supplemented with physical observations and tests which can only be done at the clinic 20 and not monitored remotely. Accuracy of the data provided to the clinic 20 about a subject's activity outside of the clinic 20 and at home is important in ensuring that the medical trial receives a true representation of the subject's activity during the monitored period. This can help to determine the efficacy of the clinical trial's therapies.

In another example, the subject data provided to the central server 140 comprises the identification data for the subject together with the accelerometer data, so that the central server 140 processes the accelerometer data for each subject from which the central server 140 works out the walking data for each subject itself. Although not illustrated in Figure 1B, in this case, the central server 140 further comprises a user interface including a user input device such as a keyboard, and/or software for processing the data collected from the user devices of the system.

The subject data indicating activity of the subject, such as walking data, is a good indicator of health or fitness levels of the subject. For example, it can be used as an indicator of recovery because step count is an indicator of general health. An increase in step count shows increased mobility, which can indicate a patient's improvement, whilst a decrease or stagnation of step count could indicate that a patient is not responding to treatment or is not showing an improvement, or even that a patient is getting more ill. An increase in step count during time periods when the treatment's effects are greatest compared to when the effect of the treatment has worn off may give an indication of the efficacy of the treatment. In some cases, step count may be indicative of a need for a patient to be called into the clinic or could indicate that the patient may be required to spend a short amount of time in hospital. In some examples, the collected patient data may be used by the clinic to help book appointments for the patient with a doctor or clinician as required.

The walking data provided by the user devices can also be used to provide one or more of the plurality of subjects 30a-30e with personalised exercise plans, tailored to their individual needs and or capabilities as indicated by the data. Prompts may be sent to a subject to encourage them to be active if their step count is too low.

Walking data is particularly useful to study in patients having one or more medical conditions which are known to affect walking capabilities. In some cases, temporary gait or balance complications may be caused by injury, trauma, inflammation or pain. In other cases, problems with walking such as gait, balance and coordination can be caused by specific conditions. Some of the conditions which may be particularly important in measuring walking activity include but are not limited to: arthritis, multiple sclerosis (MS), Meniere's disease, brain damage for example caused by a haemorrhage or tumour, Parkinson's disease, orthopaedic surgery on hips or lower body, cancer and associated therapies, cerebral palsy, obesity, gout, muscular dystrophy, stroke, spinal injury, deformities, etc.

The step and stride count data can also be used for athletic performance measurement and management. Detailed analysis of step and stride counts during targeted assessments of athletic activities can be provided to the athletes or their trainers and coaches. That data can then be used to inform training regimens to improve athletic performance.

The step and stride count data can also be used for physical therapy performance measurement and management. Detailed analysis of step and stride counts during targeted assessments during managed or unmanaged therapy sessions activities can be provided to the patient or their therapists and doctors. That data can then be used to inform therapeutic regimens to improve recovery programs.

### User Device

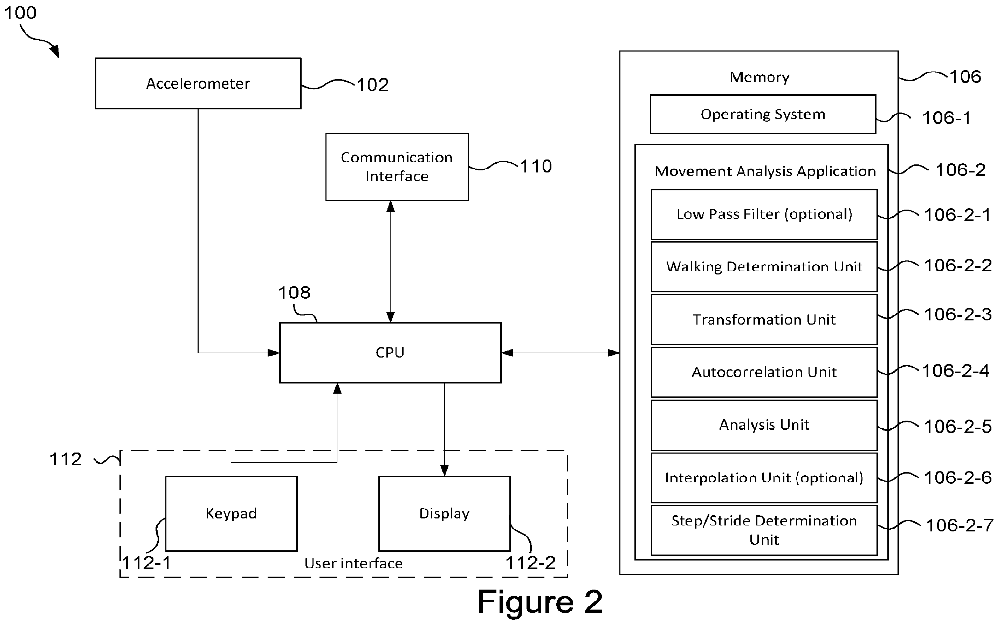

Figure 2 is a block diagram of a typical user device 100 that is used in the system described above. As shown, in this case, the user device 100 has an accelerometer 102 that provides accelerometer data to at least one central processing unit (CPU) 108. The operation of the CPU 108 is controlled by software instructions that are stored in memory 106. As shown, the software instructions include an operating system 106-1 and a movement analysis application 106-2. The accelerometer data from the accelerometer 102 is processed by the movement analysis application 106-2 to work out the walking data for the subject.

The user device 100 also includes a communication interface 110 for communicating the subject data determined by the movement analysis application 106-2 to the central server 140; and a user interface 112 comprising a keypad 112-1 and a display 112-2 to allow the subject to interact with the user device 100. The display 112-2 may display one or more icons configured to provide information to the user and/or one or more of: time, date, number of steps, activity specific icons (walking running, cycling, etc.), activity duration, reminder messages and/or instructions concerning activity, network connection status, remaining battery power and any other useful information to be displayed to the user.

### Accelerometer Data Analysis

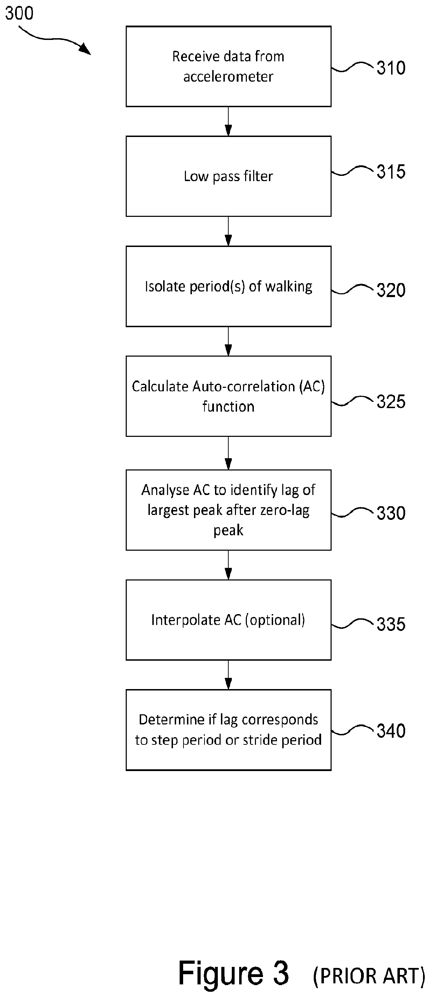

Before describing the way in which the movement analysis application 106-2 processes the accelerometer data, a description will be given with reference to Figure 3 of the conventional way in which fitness trackers and the like process accelerometer data to determine steps taken by the user.

In step 310, data from the accelerometer 102 is received. The accelerometer data comprises a series of data points indexed by time, with the data point (reading) from the accelerometer at time t comprising acceleration measurements (A_{Ax}(t), A_{Ay}(t), A_{Az}(t)) in the three orthogonal directions: A*x*, A*y* and A*z* that are aligned with (defined by) the orientation of the accelerometer 102, rather than the orientation of the person carrying the accelerometer or any other geographic coordinate system. Readings from the accelerometer 102 are typically provided in units of *g,* where *g* is acceleration due to gravity at the Earth's surface (9.8 m/s²). Sampling rates (the rate at which the accelerometer 102 provides the acceleration readings) will vary between accelerometers and are often configurable, but to be useful for analysing walking, the sampling rate should be at least 20 Hz, preferably higher (e.g. 30 Hz or 100 Hz).

Upon receiving the accelerometer data, conventional devices low pass filter the data to remove high frequency variations in the accelerometer data that are unrelated to walking movement of the user. The low pass filter will typically have a cut-off frequency of about 10 Hz. In step 320, the time series accelerometer data is processed to identify periods of walking from other periods in which the user is not walking. There are various methods by which this determination can be made. Typically, the conventional way to isolate periods of walking from other periods is to compare the magnitude of the accelerometer data with a threshold to identify periods of activity which may correspond to walking. The magnitude of the acceleration data provided at time *t* may be calculated as follows: A_{mag}(t) = sqrt*( Aa_{X}(t)² + A_{Ay}(t)² + A_{Az}(t)²* )*.* Periods thus identified are then analysed to determine if their periodic patterns correspond to those of walking (i.e. are consistent with typical stride or step periods). In step 325, an autocorrelation analysis is performed to detect periodic patterns in the time series magnitude data calculated during step 320. Specifically, the autocorrelation unit 106-2-3 calculates the autocorrelation of the time series magnitude data M(t) obtained in each isolated period of walking (or section of walking) that is identified in step 320. That is the autocorrelation unit 106-2-3 calculates: $AC \left(k\right) = {\sum }_{n = 1}^{T - k} {A}_{mag} \left(n\right) . {A}_{mag} \left(n-k\right)$

Where AC(k) is the autocorrelation at lag k; A_{mag}(n) is the accelerometer magnitude at time n within the isolated period (section) of walking; and T is the number of magnitude values within the isolated period of walking. The auto-correlation function of each period of walking is calculated. Thus, if step 320 isolates twenty periods of walking, then in step 325, the autocorrelation unit 106-2-3 calculates twenty autocorrelation functions - one for each isolated period of walking.

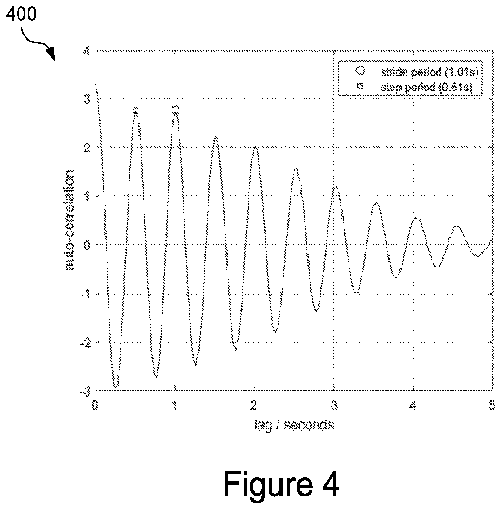

In step 330, each autocorrelation function that is calculated in step 325 is analysed to determine the lag where the highest peak after the zero-lag peak is to be found in the autocorrelation function. The calculated lag corresponds to either the user's stride period or the user's step period. To illustrate this analysis, Figure 4 is a plot representing the autocorrelation function that is determined in step 325 for one of the isolated periods (sections) of walking. The auto-correlation function is symmetric about zero-lag (k=0) and only the part corresponding to non-negative lags is shown in the plot. The peaks corresponding to the stride and step periods are marked with a circle and a square respectively. The typical stride period is between 1.0 and 1.2 seconds (100-120 steps per minute) and the typical step period will be half of this value.

The part of the autocorrelation function calculated between zero-lag and the first point at which the autocorrelation function is less than zero is considered to be the zero-lag peak. The lag of the highest peak in the autocorrelation function after the zero-lag peak is taken to be either the step or the stride period. As the autocorrelation function is calculated at a plurality of defined lags, the autocorrelation values that are calculated may not include the autocorrelation value exactly at the peak. A potentially more accurate estimate for the lag corresponding to the peak in the autocorrelation function can be determined using interpolation. This can be achieved, for example, by fitting a second-order polynomial to the calculated peak value and its neighbour on either side, and taking the peak of the polynomial function as the peak of the autocorrelation function to work out a more accurate value of the lag corresponding to the highest peak.

In step 340, a determination is made as to whether the lag corresponding to the identified highest peak corresponds to a step period or a stride period of the user. Depending on how symmetric the user's gait is and also the wear position of the accelerometer, the lag calculated in step 330 (or 335) may correspond to either the step or the stride period. For example, assuming the subject's gait is symmetric, if the accelerometer is worn/held centrally to the user's body, e.g. a phone held in front of the chest, or a device attached to the small of the user's back, then a left step and a right step will produce very similar magnitudes of acceleration at the accelerometer and the lag that is calculated is likely to correspond to the step period. On the other hand, if the accelerometer is attached to an ankle or wrist, then the left and right steps may result in substantially different acceleration data and the lag that is calculated is likely to correspond to the stride period.

In the example autocorrelation function illustrated in Figure 4, the peak in the autocorrelation function at a lag of 0.5 seconds is almost as high as the peak in the autocorrelation function at a lag of 1.0 seconds and a slight variation in the accelerometer data might change which peak is the highest and therefore which peak is identified as the highest peak in step 330.

To determine whether the highest peak found in the autocorrelation function corresponds to the stride period or the step period, conventional fitness devices compare the determined lag with a threshold value. For a particular individual at a particular moment in time, the step period will be half the stride period (assuming the right step period and the left step period are identical). Therefore, if the lag that is found is below the threshold value (e.g. 0.8 seconds), then it can be assumed that the highest peak corresponds to the step period; and if the lag is found to be above the threshold, then it can be assumed that the highest peak corresponds to the stride period.

The determined step/stride period that is calculated for each of the isolated periods of walking is then used to calculate various characteristics of the user's walking - such as the number of steps taken, the length of time the user has walked etc. and this information is output (typically displayed) to the user and/or to a central server.

However, across a given population there will be an overlap between stride and step periods: the step period of some individuals might be longer than the stride period of others. Therefore, using thresholding to try to determine if a calculated lag period corresponds to a step period or a stride period is imperfect and will lead to errors. Calibration of the user device to the individual carrying the device or providing additional knowledge about the individual (e.g. their height) may help to reduce these errors. However, even for a particular individual there may be overlap between their stride and step periods, depending on their gait at any moment in time (e.g. running vs walking). Thus, in a material proportion of cases, using a threshold calculation to determine if a calculated lag period corresponds to a step period or a stride period will result in the wrong conclusion and this will affect the accuracy of the step counts that are obtained. For example if it is determined that the calculated lag period corresponds to the step period whilst in reality it corresponds to the stride period, then the number of steps calculated will be half the true value, which is likely to have knock-on effects on the estimations of other parameters, such as speed and distance travelled. Conversely if it is determined that the calculated lag period corresponds to the stride period whilst in reality it corresponds to the step period, then the number of steps calculated will be twice the true value.

### Measurement Analysis Application

The measurement analysis application 106-2 has been developed to at least reduce some of these errors with conventional systems and to determine more accurate step and/or stride information from the accelerometer data. The way in which the measurement analysis application 106-2 operates in this embodiment will now be described in detail.

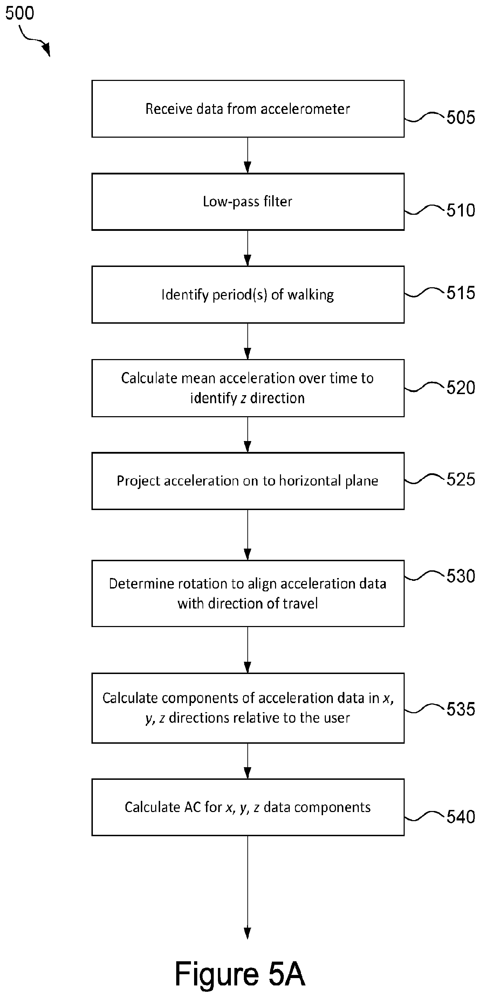

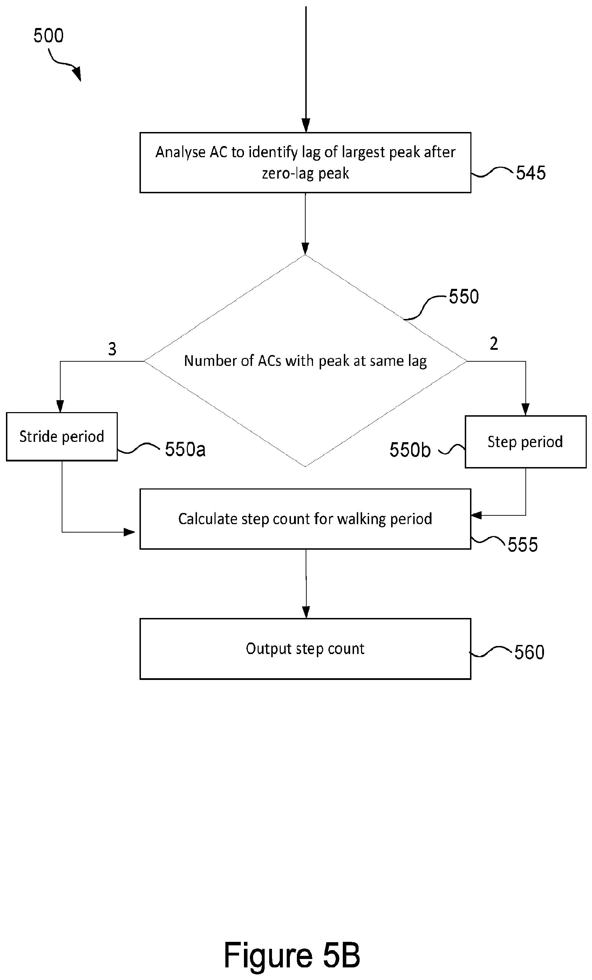

Referring to Figures 2 and 5, the measurement analysis application 106-2 receives, in step 505, the time series measurement data from the accelerometer 102. As discussed above, the acceleration data from the accelerometer at time t is defined as (A_{Ax}(t), A_{Ay}(t), A_{Az}(t)), with the accelerations being calculated along the three orthogonal directions: A*x*, A*y* and A*z* that are defined by the orientation of the accelerometer 102. Thus, each acceleration data point effectively defines a vector that defines the resulting direction of the acceleration experienced by the accelerometer 102 at the measurement time t. An optional low pass filter 106-2-1 filters the time series measurement data points received from the accelerometer in step 510, to remove high frequency variations in the accelerometer measurements that are not associated with walking movement of the user. The cut off frequency of the low pass filter is typically between 8Hz and 20Hz and preferably about 10Hz.

In step 515, the walking period detection unit 106-2-2 processes the accelerometer data to detect periods when the user is walking or running. As discussed above, there are various ways that these periods can be detected. In a typical situation, an isolated period of walking will be about 10 to 20 seconds long. If longer time periods of walking are detected, these longer periods are usually split into sections, each of which is typically 10 to 20 seconds long.

A transformation unit 106-2-3 then processes the accelerometer data to project the measurements on to a co-ordinate reference frame defined by the orientation of the user walking - specifically so that z-axis is aligned vertically, the y-axis is aligned with the direction in which the user is travelling and the x-axis is aligned with the horizontal direction transverse to the direction of travel. In this embodiment, this is achieved in the following manner:
1) The mean acceleration vector over a period of time (several seconds) is determined in step 520: ${A}_{mean} = 1 / N {\sum }_{n = 1}^{N} A \left(n\right)$ Where A(n) is the accelerometer data point at time n; N is defined by the sample rate of the accelerometer and the period of time over which the mean is computed. Gravity is the largest static component of acceleration measured by the accelerometer 102. The other accelerations experienced by the accelerometer will include accelerations in the forwards and backwards and side to side directions which to some extent cancel each other out when averaged over time. As a result, the mean vector calculated in step 520 identifies the vertical direction.
2) In step 525, the transformation unit 106-2-3 uses the determined mean vector to perform a first transformation that projects each acceleration data point from the accelerometer (A(t) - after low pass filtering if performed) onto the horizontal plane as follows: ${A}^{proj} \left(t\right) = A \left(t\right) - \left(A\left(t\right)\cdot {{A}_{mean}}^{U}\right) {{A}_{mean}}^{U}$ Where Aₘₑₐₙ^{U} is the unit vector of the mean acceleration vector determined in step 520. Whilst the z-axis of the resulting projected data points aligns with the vertical axis, the projected y-axis of the accelerometer is unlikely to align with the direction of travel (forwards and backwards direction) and the projected x-axis of the accelerometer is unlikely to align with the direction transverse to the direction of travel (side to side direction).
3) In step 530, the transformation unit 106-2-3 effectively works out the rotation needed to be applied to the projected acceleration data in order to align the projected x- and y-axes of the accelerometer with the desired side to side direction and forwards/backwards direction respectively. This rotation angle can be found in different ways. In this embodiment, the transformation unit 106-2-3 performs a principal component analysis (PCA) on the projected data (after setting the z-axis values in the projected data points to zero). The PCA analysis will identify the two orthogonal directions in the horizontal plane that have the most and the least variability. The direction with most variability will usually correspond to the movements in the forwards/backwards direction (y-direction) and the direction with least variability will usually correspond to the movements in the side to side direction (x-direction). The orthogonal directions identified by the PCA analysis effectively define the rotation within the horizontal plane that needs to be applied to the projected data points in order to align the projected x- and y-axes of the accelerometer with the desired side to side direction and forwards and backwards direction respectively.
4) In step 535, the transformation unit 106-2-3 applies the rotation determined in step 530 to the projected accelerometer data obtained in step 525. This generates, for the accelerometer data at time t, a transformed acceleration data point: Aᵣₒₜ^{proj}(t) that identifies the acceleration in the vertical direction (the z-axis), the acceleration in the forwards-backwards direction (y-axis) and the acceleration in the side to side direction (x-axis).

In step 540, the autocorrelation unit 106-2-4 calculates the autocorrelation function of the vertical acceleration data (z-axis data), an autocorrelation of the forwards-backwards acceleration data (y-axis data) and an autocorrelation of the side to side acceleration data (x-axis data), for each of the isolated walking periods identified by the walking period determination unit 106-2-1. That is the following autocorrelations are calculated: ${AC}_{z} \left(k\right) = {\sum }_{n = 1}^{T - k} {Az}_{rot}^{proj} \left(n\right) . {Az}_{rot}^{proj} \left(n-k\right) mag$ ${AC}_{y} \left(k\right) = {\sum }_{n = 1}^{T - k} {Ay}_{rot}^{proj} \left(n\right) . {Ay}_{rot}^{proj} \left(n-k\right) mag$ ${AC}_{x} \left(k\right) = {\sum }_{n = 1}^{T - k} {Ax}_{rot}^{proj} \left(n\right) . {Ax}_{rot}^{proj} \left(n-k\right) mag$ where ${Az}_{rot}^{proj} \left(n\right)$ is the acceleration data in the vertical direction at time point n; ${Ay}_{rot}^{proj} \left(n\right)$ is the acceleration data in the forwards-backwards direction at time point n; ${Ax}_{rot}^{proj} \left(n\right)$ is the acceleration data in the side to side direction at time point n; k is the autocorrelation lag; and T is the number of acceleration data points within the isolated walking period.

It should be noted that the processing above may result in the x and y data being switched over - that is the y-axis data may actually correspond to the side to side acceleration measurements and the x-axis data may correspond to the forwards/backwards acceleration measurements. However, this does not matter - as will become evident from the following discussion.

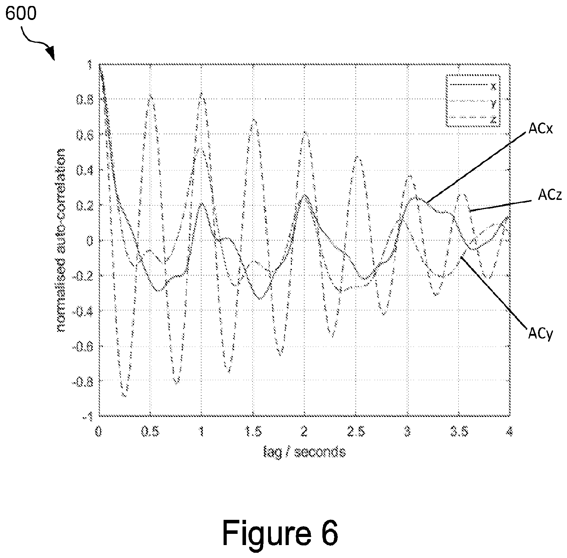

Figure 6 illustrates a plot of the three autocorrelation functions that are calculated for one of the isolated walking periods for lags (k) between 0 and 4 seconds. The autocorrelation function in the z (vertical) direction (AC_{z}) is generally much larger than the autocorrelation function in the x (side to side) direction (ACₓ) and the autocorrelation function in the y (forwards/backwards) direction (AC_{y}), so each autocorrelation function shown in Figure 6 has been scaled to be unity at zero lag for ease of comparison.

As can be seen from Figure 6, the autocorrelation function for the *z* direction(AC_{z}), has a similar plot to the autocorrelation of the magnitude of the original acceleration data (as shown in Figure 4) and has strong peaks at both 0.5 seconds (the step period) and at 1.0 seconds (the stride period). Again, however, the highest peak in AC_{z} after the zero-lag peak may correspond to the step period or to the stride period. The autocorrelation function for both the *x* and *y* directions (ACₓ and AC_{y}) also have noticeable peaks at 1.0 second (the stride period). The data used to generate the example autocorrelation functions shown in Figure 6 were obtained from a wrist-worn user device and the peak in the autocorrelation function AC_{y} at the step period is present, but subdued. For centrally worn devices, the peak in the autocorrelation function AC_{y} at the step period would be more pronounced. However, the autocorrelation function ACₓ lacks a peak at 0.5 seconds (the step period) regardless of how the user device is carried or worn.

**Table 1 below summarises whether there is likely to be a peak in the autocorrelation functions for the vertical (AC_{z}), forwards-backwards (AC_{y}) and side-to-side (ACₓ) directions at lags corresponding to the stride and step periods for different wear positions of the user device/accelerometer.**

| Wear position | AC lag | Direction | | |
|---|---|---|---|---|
| | | Vertical AC_{z} | Forwards-backwards AC_{y} | Side-to-side ACₓ |
| ankle / wrist | stride period | Yes | yes | yes |
| ankle / wrist | step period | Yes | maybe | no |
| central | stride period | Yes | yes | yes |
| central | step period | Yes | yes | no |

ACx is likely to exhibit a trough at the step period. The lack of a peak in ACₓ at the step period can be used to distinguish between whether the highest peak in AC_{z} after the zero-lag peak corresponds to the stride period or the step period - without having to use thresholds.

Specifically, in step 545, the analysis unit 106-2-5 processes the autocorrelation values AC_{z} obtained for the vertical direction to identify the lag corresponding to the largest peak after the zero-lag peak. As before, the optional interpolation unit 106-2-6 may use interpolation using a polynomial function to determine a more accurate estimate of the lag corresponding to this largest peak. Then, in step 550, the step/stride determination unit 106-2-7 determines if the autocorrelation functions for the x and y directions (ACₓ and AC_{y}) also have peaks at the lag identified in step 545. If both ACx and ACy also have peaks at (or around) this lag, then the step/stride determination unit 106-2-7 determines that the lag identified in step 545 corresponds to the stride period of the user. However, if only one (or neither) of ACx and ACy have a peak at the identified lag, then the step/stride determination unit 106-2-7 determines that the lag identified at step 545 corresponds to the user's step period. There are various different ways for determining if ACx or ACy exhibit a "peak": often there will be a peak (i.e. a sample of the auto-correlation that is higher than its neighbours on either side) at or near the lag identified in step 545. In other embodiments, if ACx or ACy at the determined lag is above a threshold, which may be zero or may be relative to the autocorrelation at zero lag, it is deemed to be a peak. It should be noted that this approach does not rely on the assumption that y corresponds to the forwards-backwards direction and x corresponds to the side-to-side direction; this approach is still valid if x corresponds to the forwards-backwards direction and y corresponds to the side-to-side direction.

Once the step/stride determination unit 106-2-7 has determined if the lag identified at step 545 corresponds to the user's step period or the user's stride period, the movement analysis application 106-2 can calculate in step 555 the number of steps taken by the user during the walking period. This information is then output in step 560. The step count may be output to the user on the display 112-2 and/or it may be transmitted together with other related walking data and an identifier to identify the user to whom the data relates to the central server 140 for use in the clinical trial.

### Modifications and Variations

A detailed embodiment has been described above. Various modifications and changes can be made to the above embodiment. Some of these variations will now be described.

In the embodiment described above, there is an implicit assumption that the orientation of the accelerometer 102 remains the same (constant) within each of the isolated periods of walking determined in step 515 and over which the autocorrelation functions are calculated. It also assumes that the characteristics of the walking (in particular the step/stride periods) are relatively constant over the isolated period of walking. These assumptions may not be correct, especially for longer isolated periods of walking. To address this issue, the isolated walking periods may be divided into smaller subsections or epochs (that may or may not overlap in time) with the above analysis from step 520 then being performed on each smaller subsection of accelerometer data. The duration of each subsection should be at least 3 seconds long in order to encompass a number of strides. When the rotation is calculated in step 530 for a subsection, the PCA analysis may cause the determined rotation to change abruptly from one subsection to the next. Interpolation may be used (e.g. using the quaternion representation or other means) to provide a smooth transition between the rotations of adjacent subsections. Data from other sensors (in particular gyroscopes that may also be mounted in the user device) may also be useful to determine changes in orientation of the user device - and hence the changes in rotation required to align the acceleration data with the direction of travel of the user.

In the above embodiment, measurements from an accelerometer were resolved into a vertical direction (z) and into x and y directions corresponding to the user's direction of travel and side to side direction. Autocorrelation functions were then calculated for the measurements in the x, y and z directions. In an alternative embodiment, instead of determining the autocorrelation function of the acceleration data in the z-direction, the autocorrelation may be performed on the magnitude of the accelerometer data (either before or after transformation). The autocorrelation functions in the x and y directions would still be calculated and used as before in order to resolve the ambiguity over whether the highest peak after the zero-lag peak in the autocorrelation function of the magnitude data corresponds to the user's stride period or the user's step period.

In the above embodiment, the analysis unit 106-2-5 used a principal component analysis to work out the rotation needed to align the projected x and y axis of the accelerometer onto the walking direction and side to side direction of the user. Instead of using PCA to determine this rotation, a satellite navigation system (such as a GPS system) provided in the user device may provide the geographical direction that the user is walking in and a compass in the user device may provide the orientation of the device relative to the geographic axes and from this the analysis unit 106-2-5 can work out the rotation needed to map the acceleration data from the accelerometer onto the reference frame of the user walking (with y corresponding to the direction that the user is walking in, with x being transverse to y in the horizontal plane and with z being the vertical direction).

Alternatively if the device is at a fixed, known orientation to the user's direction of travel - for example if the device is held pointing in the direction of travel - then the rotation needed to map the acceleration data from the accelerometer onto the reference frame of the user may already be known.

The x, y, z autocorrelation functions calculated in the above embodiment may also be useful for distinguishing between walking and other activities. For example, walking detection algorithms can be tricked by a user with a wrist-worn user device swinging their arm - if the swing period is similar to a typical stride period, then the arm-swinging may be wrongly interpreted as walking. The autocorrelation data determined in the x, y, z directions can be used to confirm that a period of walking is actually a period of walking rather than the user moving the device to try to mimic walking movements.

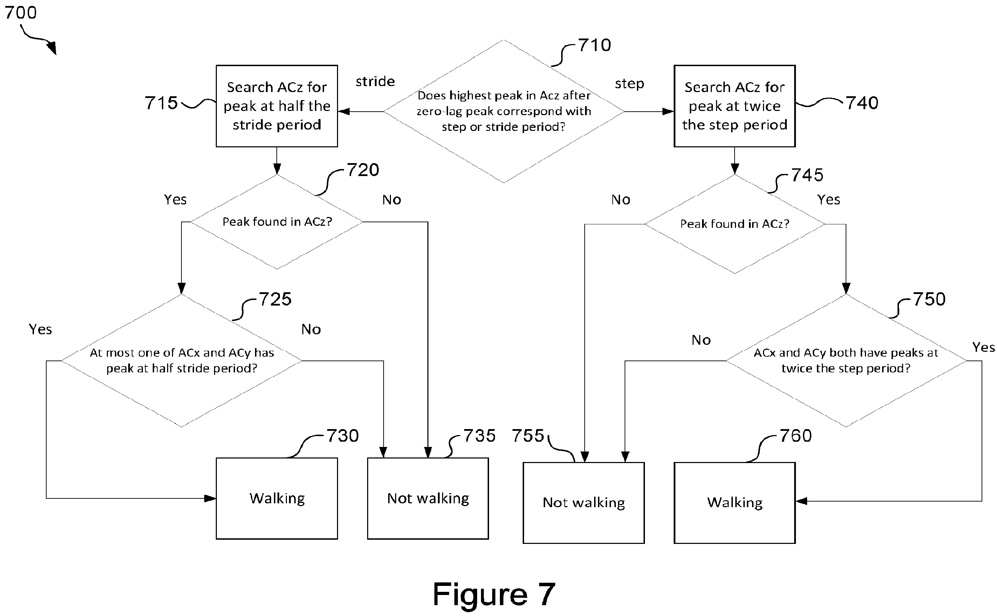

Figure 7 is a flow diagram illustrating the way in which the system can determine more accurately whether a period of movement corresponds to a period of walking or to some other user movement that is trying to mimic walking.

In step 710, the device determines if the highest peak after the zero-lag peak in AC_{z} (or in the autocorrelation of the magnitude accelerometer data) corresponds to the user's stride or step period (this effectively corresponds to the determination made at step 550 or 555 in Figure 5). If the highest peak corresponds to the user's stride period, the process proceeds to step 715 and if the highest peak corresponds to a step period, the process proceeds to step 740.

At step 715 the walking determination unit 106-2-2 processes the autocorrelation function for the z (vertical) direction (AC_{z}) to determine if it has a peak at a lag that is half the lag corresponding to the stride period. At step 720 if a peak is found in AC_{z} at half the stride period, the process proceeds to step 725. If a peak is not found in AC_{z} at half the stride period, then the walking determination unit 106-2-2 determines at step 735 that the user is not actually walking in this period.

At step 725, the walking determination unit 106-2-2 checks the autocorrelation functions for the x and y directions (ACₓ and AC_{y}) to determine if at most one of ACₓ and AC_{y} also contains a peak at half the stride period. If they both contain a peak at half the stride period, then the processing proceeds to step 735 where the walking determination unit 106-2-2 determines again that the movement in the current period is not actually walking. If neither or only one of ACₓ and AC_{y} has a peak at half the stride period, then the processing moves to step 730 where the walking determination unit 106-2-2 confirms that the user is actually walking in the current period.

If the highest peak after the zero-lag peak in AC_{z} corresponds to the user's step period, then in step 740 the walking determination unit 106-2-2 processes the autocorrelation function for the z (vertical) direction (AC_{z}) to determine if it has a peak at twice the step period. If a peak is not found in AC_{z} at twice the step period, then the walking determination unit 106-2-2 determines at step 755 that the user is not actually walking in this period.

At step 750, the walking determination unit 106-2-2 checks the autocorrelation functions for the x and y directions (ACₓ and AC_{y}) to determine if both of them also contain a peak at twice the step period. If they do not both contain a peak at twice the step period, then the processing proceeds to step 755 where the walking determination unit 106-2-2 determines again that the movement in the current period is not actually walking. If both of ACₓ and AC_{y} have a peak at twice the step period, then the processing moves to step 760 where the walking determination unit 106-2-2 confirms that the user is walking in the current period.

In the above embodiment, the step/stride determination unit considered the presence and absence of peaks in the forwards/backwards direction (y direction) and in the side to side direction (x direction), to disambiguate whether the lag period of the highest peak after the zero-lag peak corresponds to the step period or the stride period. The preferred technique counted peaks in the different autocorrelation functions at the same lag. This helps to avoid any errors where the forwards/backwards direction is mixed up with the side to side direction. In other embodiments, the device analysing the accelerometer data may simply assume that the determined side to side direction (x direction) is correct, and then may disambiguate whether the identified lag corresponds to the step period or the stride period in dependence upon whether the autocorrelation function for the x direction includes a peak at the identified lag. If it does, then it is the stride period and if it does not then it is the step period.

In the above embodiments, the accelerometer data obtained from the accelerometer was analysed by looking at the autocorrelation function of the data in the different directions. The autocorrelation analysis is good at highlighting periodic changes in the acceleration data - caused by repetitive movements such as walking and running. Other kinds of analysis could be performed to identify these periodic changes (and the period thereof). For example, a Fourier Transform (or other frequency analysis such as a Discrete Cosine Transform) could be determined and analysed to identify peaks in the frequency domain representative of the step or stride period.

Similarly, in the above embodiment, the acceleration data from the accelerometer is transformed from the co-ordinate reference frame of the accelerometer to the co-ordinate reference frame of the user and then the autocorrelation was performed on the transformed acceleration data. In an alternative embodiment, this transformation of the co-ordinate system may happen after the autocorrelation functions have been calculated. Thus, the original accelerometer data defining the accelerations of the accelerometer in directions Ax, Ay and Az may be subject to an autocorrelation analysis first and then the autocorrelations are transformed to account for the change in reference frame.

Further, in the case that the user's device has multiple accelerometers built into it, the data from each accelerometer may be analysed and the results combined (for example averaged) to work out more accurate or less noisy step and/or stride periods. Similarly, where the user is carrying multiple devices (such as a cellular telephone) and an actigraph device, where both devices have an accelerometer, the system can determine step and/or stride periods using the data from both accelerometers. The measurements from the two (or more) accelerometers can then be averaged again to improve signal to noise ratio or the measurements from one accelerometer may be used to corroborate or validate the step and/or stride period determined from acceleration data obtained from the other accelerometer.

As mentioned above, the peaks in the autocorrelation functions calculated depend on whether the accelerometer is worn or carried centrally to the user or is worn or carried on the user's wrist or ankle. The inventors have realised that by comparing the amplitudes of the peaks in the autocorrelation function at the step and stride lags, it is possible to determine how the user is holding or wearing the accelerometer. Specifically, the user's device or the central server can compare the peak values of the autocorrelation function at lags corresponding to the step period and the stride period (which may be determined using the invention described above or using other prior art techniques) and if the two peaks are the same or similar in value to each other or if the peak at the step period is larger than the peak at the stride period, then the user device or the central server can determine that the accelerometer is being worn or carried centrally. The inventors have found that in this situation the peaks can be considered to be similar in value to each other if they are within 10% to 15% (or less) of each other. However, if the peak at the stride period is 20% or more larger than the peak at the step period, then the user device or the central server can determine that the accelerometer is likely to be being worn or carried on the user's periphery (such as in their hand or on their wrist or ankle). Similarly, if a frequency analysis is performed on the acceleration data instead of an autocorrelation analysis, a comparison of the magnitudes of the peaks in the frequency plots at frequencies corresponding to the step and stride frequencies can be used to determine if the accelerometer is being worn/carried centrally or peripherally (for example on the user's wrist or ankle). Knowing if the accelerometer device is carried centrally or peripherally is useful as knowledge of this information can help to interpret the acceleration data and adjust thresholds that are used. For example, if the acceleration data is being used to determine an activity level for the user, then different thresholds may be used depending on whether the device is being carried centrally or peripherally. In particular, if a user is sedentary at a desk writing or typing, a device worn on a wrist will likely experience more movement than one worn on a belt and this difference would need to be accounted for if the central server is trying to determine how active the user is. Although the wear position may change, it is reasonable to assume that a wear position determined whilst the user is walking will be the same on the same day when the user is perhaps sedentary. Additionally, some algorithms that are used to determine the distance walked and other gait parameters (such as parameters to characterise freezing-of-gait in Parkinson's patients) assume that the accelerometer device is ankle worn and will be less accurate or will need recalibration if the device is centrally worn. Therefore, knowledge of the wear position of the accelerometer device allows for such recalibrations to be made, resulting in improved consistency and accuracy of the measures and in the alternative allows the data to be acknowledged as less accurate.

The wear position of the accelerometer is determined using the autocorrelation function (or frequency function) on the original acceleration data (or components thereof) or on the magnitude of the acceleration data. If the techniques described above are used to resolve the acceleration data into the user's reference frame (vertical, side to side and forwards and backwords), then the wear position of the accelerometer can also be determined by comparing the dominant period of the lateral (side to side) acceleration with the dominant period of the vertical component of the acceleration data or of the magnitude of the acceleration. If the dominant periods are the same (or are within a level of acceptable tolerance of each other), then the user's device or the central server can determine that the accelerometer is most likely being worn on/carried by a limb of the user (e.g. on their wrist or ankle). The inventors have found that an acceptable level of tolerance in this case is within 10 to 15% of each other. The dominant period is the lag corresponding to the largest peak of the autocorrelation function (after the zero lag peak) and in this scenario it will correspond to the stride period.) A similar analysis can be performed on the frequency plots of the resolved acceleration data.

However, if the dominant period of the lateral acceleration is about twice the dominant period of the vertical acceleration component or the acceleration magnitude, and the lateral acceleration component has little or no energy at the dominant period of the vertical acceleration component or the acceleration magnitude, then the user's device or the central server can determine that the accelerometer is most likely being carried in/worn on a more central body position e.g. on the user's chest or waist. (In this scenario the dominant period of the lateral component will be at the stride period, whereas the dominant period of vertical/magnitude will be at the step period.)

In the above embodiment, a software application for processing accelerometer data was provided in the user device. The same or similar software may be provided in the computer of the central server - so that the central server performs the above step/stride analysis. This software application may be provided as computer implementable instructions on a carrier signal or on a tangible computer readable medium. Alternatively, the functions of the software application may be defined in hardware circuits such as in FPGA or ASIC devices.

It will be appreciated from the above description that many features of the different examples are interchangeable and combinable. The disclosure extends to further examples comprising features from different examples combined together in ways not specifically mentioned. Indeed, there are many features presented in the above examples and it will be apparent to the skilled person that these may be advantageously combined with one another.

In the above embodiments, various lags or periods were calculated. As those skilled in the art will appreciate, the determination of such periods will happen if corresponding frequencies are determined. Specifically, in the above embodiments, the step period and the stride period are determined. Clearly, if a step frequency or a stride frequency is determined, this implicitly also means that a corresponding step or stride period is determined as well and the claims are intended to cover the determination of such frequencies instead of or in addition to said periods/lags.

## Claims

1. An apparatus for determining movement information for a user that carries an accelerometer (102) whilst moving, the apparatus comprising one or more processors (108) and memory (106) configured to:
receive (505) acceleration data from the accelerometer (102), the acceleration data defining accelerations experienced by the accelerometer (102) resulting from movement of the user, the accelerations being defined relative to a frame of reference associated with the accelerometer (102);
apply to the acceleration data or to data derived from the acceleration data a transformation (106-2-3) for transforming the frame of reference to a frame of reference of the user that includes a vertical direction, a direction of travel of the user and a side to side direction transverse to the direction of travel of the user;
determining (106-2-5) a period corresponding to a largest peak of an autocorrelation function or a frequency function of acceleration data in said side to side direction;
determining (106-2-5) a period corresponding to a largest peak of an autocorrelation function or a frequency function of acceleration data in said vertical direction or of acceleration magnitude data; the one or more processors and memory being **characterized by**
determining (106-2-5) if the accelerometer (102) is carried by the user centrally of the user's body or if the accelerometer (102) is carried by the user on a peripheral part of the user's body using the determined periods.

2. The apparatus according to claim 1, wherein the one or more processors (108) and memory (106) are configured to determine that the accelerometer (102) is carried centrally of the user's body if the period corresponding to a largest peak of the autocorrelation function or a frequency function of acceleration data in said side to side direction is about twice the period corresponding to a largest peak of the autocorrelation function or the frequency function of acceleration data in said vertical direction or of acceleration magnitude data.

3. The apparatus according to claim 1 or 2, wherein the one or more processors (108) and memory (106) are configured to determine that the accelerometer (102) is carried peripherally of the user's body if the period corresponding to a largest peak of the autocorrelation function or a frequency function of acceleration data in said side to side direction is about the same as the period corresponding to a largest peak of the autocorrelation function or the frequency function of acceleration data in said vertical direction or of acceleration magnitude data.

4. A method of determining movement information for a user that carries an accelerometer (102) whilst moving, the method comprising using one or more processors (108) and memory (106) to:
receive (505) acceleration data from the accelerometer (102), the acceleration data defining accelerations experienced by the accelerometer (102) resulting from movement of the user, the accelerations being defined relative to a frame of reference associated with the accelerometer (102);
apply to the acceleration data or to data derived from the acceleration data a transformation (106-2-3) for transforming the frame of reference to a frame of reference of the user that includes a vertical direction, a direction of travel of the user and a side to side direction transverse to the direction of travel of the user;
determining (106-2-5) a period corresponding to a largest peak of an autocorrelation function or a frequency function of acceleration data in said side to side direction;
determining (106-2-5) a period corresponding to a largest peak of an autocorrelation function or a frequency function of acceleration data in said vertical direction or of acceleration magnitude data; the method being **characterized by**
determining (106-2-5) if the accelerometer is carried by the user centrally of the user's body or if the accelerometer is carried by the user on a peripheral part of the user's body using the determined periods.

5. The method according to claim 4, wherein the method determines that the accelerometer (102) is carried centrally of the user's body if the period corresponding to a largest peak of the autocorrelation function or a frequency function of acceleration data in said side to side direction is about twice the period corresponding to a largest peak of the autocorrelation function or the frequency function of acceleration data in said vertical direction or of acceleration magnitude data.

6. The method according to claim 4 or 5, wherein the method determines that the accelerometer (102) is carried peripherally of the user's body if the period corresponding to a largest peak of the autocorrelation function or a frequency function of acceleration data in said side to side direction is about the same as the period corresponding to a largest peak of the autocorrelation function or the frequency function of acceleration data in said vertical direction or of acceleration magnitude data.

7. A computer implementable instructions product comprising computer implementable instructions for causing a programmable computer device to perform the method of any of claims 4 to 6.

## Patentansprüche

1. Gerät zum Bestimmen von Bewegungsinformationen für einen Benutzer, der einen Beschleunigungsmesser (102) trägt, während er sich bewegt, wobei das Gerät einen oder mehrere Prozessoren (108) und Speicher (106) umfasst, ausgelegt zum:
Empfangen (505) von Beschleunigungsdaten von dem Beschleunigungsmesser (102), wobei die Beschleunigungsdaten Beschleunigungen definieren, die der Beschleunigungsmesser (102) erfährt und die aus der Bewegung des Benutzers resultieren, wobei die Beschleunigungen relativ zu einem dem Beschleunigungsmesser (102) zugeordneten Bezugsrahmen definiert werden;
Anwenden, auf die Beschleunigungsdaten oder auf von den Beschleunigungsdaten abgeleitete Daten, einer Transformation (106-2-3) zum Transformieren des Bezugsrahmens in einen Bezugsrahmen des Benutzers, der eine vertikale Richtung, eine Fortbewegungsrichtung des Benutzers und eine Richtung von Seite zu Seite quer zu der Fortbewegungsrichtung des Benutzers beinhaltet;
Bestimmen (106-2-5) einer Periode, die einem größten Spitzenwert einer Autokorrelationsfunktion oder einer Frequenzfunktion von Beschleunigungsdaten in der Richtung von Seite zu Seite entspricht;
Bestimmen (106-2-5) einer Periode, die einem größten Spitzenwert einer Autokorrelationsfunktion oder einer Frequenzfunktion von Beschleunigungsdaten in der vertikalen Richtung oder von Beschleunigungsgrößedaten entspricht; wobei der eine oder die mehreren Prozessoren und der Speicher **gekennzeichnet sind durch**
Bestimmen (106-2-5), ob der Beschleunigungsmesser (102) von dem Benutzer zentral am Körper des Benutzers getragen wird oder ob der Beschleunigungsmesser (102) von dem Benutzer an einem peripheren Teil des Körpers des Benutzers betragen wird, unter Verwendung der bestimmten Perioden.

2. Gerät nach Anspruch 1, wobei der eine oder die mehreren Prozessoren (108) und der Speicher (106) dazu ausgelegt sind, zu bestimmen, dass der Beschleunigungsmesser (102) zentral am Körper des Benutzers getragen wird, wenn die Periode, die einem größten Spitzenwert der Autokorrelationsfunktion oder einer Frequenzfunktion von Beschleunigungsdaten in der Richtung von Seite zu Seite entspricht, etwa das Zweifache der Periode beträgt, die einem größten Spitzenwert der Autokorrelationsfunktion oder der Frequenzfunktion von Beschleunigungsdaten in der vertikalen Richtung oder von Beschleunigungsgrößedaten entspricht.

3. Gerät nach Anspruch 1 oder 2, wobei der eine oder die mehreren Prozessoren (108) und der Speicher (106) dazu ausgelegt sind, zu bestimmen, dass der Beschleunigungsmesser (102) peripher am Körper des Benutzers getragen wird, wenn die Periode, die einem größten Spitzenwert der Autokorrelationsfunktion oder einer Frequenzfunktion von Beschleunigungsdaten in der Richtung von Seite zu Seite entspricht, etwa gleich der Periode ist, die einem größten Spitzenwert der Autokorrelationsfunktion oder der Frequenzfunktion von Beschleunigungsdaten in der vertikalen Richtung oder von Beschleunigungsgrößedaten entspricht.

4. Verfahren zum Bestimmen von Bewegungsinformationen für einen Benutzer, der einen Beschleunigungsmesser (102) trägt, während er sich bewegt, wobei das Verfahren das Verwenden eines oder mehrerer Prozessoren (108) und von Speicher (106) zu Folgendem umfasst:
Empfangen (505) von Beschleunigungsdaten von dem Beschleunigungsmesser (102), wobei die Beschleunigungsdaten Beschleunigungen definieren, die der Beschleunigungsmesser (102) erfährt und die aus der Bewegung des Benutzers resultieren, wobei die Beschleunigungen relativ zu einem dem Beschleunigungsmesser (102) zugeordneten Bezugsrahmen definiert werden;
Anwenden, auf die Beschleunigungsdaten oder auf von den Beschleunigungsdaten abgeleitete Daten, einer Transformation (106-2-3) zum Transformieren des Bezugsrahmens in einen Bezugsrahmen des Benutzers, der eine vertikale Richtung, eine Fortbewegungsrichtung des Benutzers und eine Richtung von Seite zu Seite quer zu der Fortbewegungsrichtung des Benutzers beinhaltet;
Bestimmen (106-2-5) einer Periode, die einem größten Spitzenwert einer Autokorrelationsfunktion oder einer Frequenzfunktion von Beschleunigungsdaten in der Richtung von Seite zu Seite entspricht;
Bestimmen (106-2-5) einer Periode, die einem größten Spitzenwert einer Autokorrelationsfunktion oder einer Frequenzfunktion von Beschleunigungsdaten in der vertikalen Richtung oder von Beschleunigungsgrößedaten entspricht; wobei das Verfahren **gekennzeichnet ist durch**
Bestimmen (106-2-5), ob der Beschleunigungsmesser von dem Benutzer zentral am Körper des Benutzers getragen wird oder ob der Beschleunigungsmesser von dem Benutzer an einem peripheren Teil des Körpers des Benutzers betragen wird, unter Verwendung der bestimmten Perioden.

5. Verfahren nach Anspruch 4, wobei das Verfahren bestimmt, dass der Beschleunigungsmesser (102) zentral am Körper des Benutzers getragen wird, wenn die Periode, die einem größten Spitzenwert der Autokorrelationsfunktion oder einer Frequenzfunktion von Beschleunigungsdaten in der Richtung von Seite zu Seite entspricht, etwa das Zweifache der Periode beträgt, die einem größten Spitzenwert der Autokorrelationsfunktion oder der Frequenzfunktion von Beschleunigungsdaten in der vertikalen Richtung oder von Beschleunigungsgrößedaten entspricht.

6. Verfahren nach Anspruch 4 oder 5, wobei das Verfahren bestimmt, dass der Beschleunigungsmesser (102) peripher am Körper des Benutzers getragen wird, wenn die Periode, die einem größten Spitzenwert der Autokorrelationsfunktion oder einer Frequenzfunktion von Beschleunigungsdaten in der Richtung von Seite zu Seite entspricht, etwa gleich der Periode ist, die einem größten Spitzenwert der Autokorrelationsfunktion oder der Frequenzfunktion von Beschleunigungsdaten in der vertikalen Richtung oder von Beschleunigungsgrößedaten entspricht.

7. Produkt mit computer-implementierbaren Anweisungen, das computerimplementierbare Anweisungen umfasst, um eine programmierbare Computervorrichtung zu veranlassen, das Verfahren nach einem der Ansprüche 4 bis 6 durchzuführen.

## Revendications

1. Appareil destiné à déterminer des informations de mouvement pour un utilisateur qui porte un accéléromètre (102) tout en se déplaçant, l'appareil comprenant un ou plusieurs processeurs (108) et une mémoire (106) configuré pour :
recevoir (505) des données d'accélération de l'accéléromètre (102), les données d'accélération définissant des accélérations rencontrées par l'accéléromètre (102) résultant du mouvement de l'utilisateur, les accélérations étant définies par rapport à un cadre de référence associé à l'accéléromètre (102) ;
appliquer aux données d'accélération ou à des données dérivées des données d'accélération une transformation (106-2-3) destinée à transformer le cadre de référence en un cadre de référence de l'utilisateur qui inclut une direction verticale, une direction de parcours de l'utilisateur et une direction de part et d'autre transversale à la direction de parcours de l'utilisateur ;
déterminer (106-2-5) une période correspondant à un pic le plus grand d'une fonction d'autocorrélation ou d'une fonction de fréquence de données d'accélération dans ladite direction de part et d'autre ;
déterminer (106-2-5) une période correspondant à un pic le plus grand d'une fonction d'autocorrélation ou d'une fonction de fréquence de données d'accélération dans ladite direction verticale ou de données d'amplitude d'accélération ; les un ou plusieurs processeurs et la mémoire étant **caractérisés par**
le fait de déterminer (106-2-5) si l'accéléromètre (102) est porté par l'utilisateur au centre du corps de l'utilisateur ou si l'accéléromètre (102) est porté par l'utilisateur sur une partie périphérique du corps de l'utilisateur en utilisant les périodes déterminées.

2. Appareil selon la revendication 1, dans lequel les un ou plusieurs processeurs (108) et la mémoire (106) sont configurés pour déterminer que l'accéléromètre (102) est porté au centre du corps de l'utilisateur si la période correspondant à un pic le plus grand de la fonction d'autocorrélation ou d'une fonction de fréquence de données d'accélération dans ladite direction de part et d'autre est environ le double de la période correspondant à un pic le plus grand de la fonction d'autocorrélation ou de la fonction de fréquence de données d'accélération dans ladite direction verticale ou de données d'amplitude d'accélération.

3. Appareil selon la revendication 1 ou 2, dans lequel les un ou plusieurs processeurs (108) et la mémoire (106) sont configurés pour déterminer que l'accéléromètre (102) est porté sur une périphérie du corps de l'utilisateur si la période correspondant à un pic le plus grand de la fonction d'autocorrélation ou d'une fonction de fréquence de données d'accélération dans ladite direction de part et d'autre est environ la même que la période correspondant à un pic le plus grand de la fonction d'autocorrélation ou de la fonction de fréquence de données d'accélération dans ladite direction verticale ou de données d'amplitude d'accélération.

4. Procédé de détermination d'informations de mouvement pour un utilisateur qui porte un accéléromètre (102) tout en se déplaçant, le procédé comprenant l'utilisation d'un ou de plusieurs processeurs (108) et d'une mémoire (106) pour :
recevoir (505) des données d'accélération de l'accéléromètre (102), les données d'accélération définissant des accélérations rencontrées par l'accéléromètre (102) résultant du mouvement de l'utilisateur, les accélérations étant définies par rapport à un cadre de référence associé à l'accéléromètre (102) ;
appliquer aux données d'accélération ou à des données dérivées des données d'accélération une transformation (106-2-3) destinée à transformer le cadre de référence en un cadre de référence de l'utilisateur qui inclut une direction verticale, une direction de parcours de l'utilisateur et une direction de part et d'autre transversale à la direction de parcours de l'utilisateur ;
déterminer (106-2-5) une période correspondant à un pic le plus grand d'une fonction d'autocorrélation ou d'une fonction de fréquence de données d'accélération dans ladite direction de part et d'autre ;
déterminer (106-2-5) une période correspondant à un pic le plus grand d'une fonction d'autocorrélation ou d'une fonction de fréquence de données d'accélération dans ladite direction verticale ou de données d'amplitude d'accélération ; le procédé étant **caractérisé par**
le fait de déterminer (106-2-5) si l'accéléromètre est porté par l'utilisateur au centre du corps de l'utilisateur ou si l'accéléromètre est porté par l'utilisateur sur une partie périphérique du corps de l'utilisateur en utilisant les périodes déterminées.

5. Procédé selon la revendication 4, dans lequel le procédé détermine que l'accéléromètre (102) est porté au centre du corps de l'utilisateur si la période correspondant à un pic le plus grand de la fonction d'autocorrélation ou d'une fonction de fréquence de données d'accélération dans ladite direction de part et d'autre est environ le double de la période correspondant à un pic le plus grand de la fonction d'autocorrélation ou de la fonction de fréquence de données d'accélération dans ladite direction verticale ou de données d'amplitude d'accélération.

6. Procédé selon la revendication 4 ou 5, dans lequel le procédé détermine que l'accéléromètre (102) est porté sur une périphérie du corps de l'utilisateur si la période correspondant à un pic le plus grand de la fonction d'autocorrélation ou d'une fonction de fréquence de données d'accélération dans ladite direction de part et d'autre est environ la même que la période correspondant à un pic le plus grand de la fonction d'autocorrélation ou de la fonction de fréquence de données d'accélération dans ladite direction verticale ou de données d'amplitude d'accélération.

7. Produit d'instructions pouvant être mises en œuvre par ordinateur comprenant des instructions pouvant être mises en œuvre par ordinateur destinées à amener un dispositif d'ordinateur programmable à réaliser le procédé selon l'une quelconque des revendications 4 à 6.
